# EUROPEAN PATENT SPECIFICATION

(11) **EP 4 408 353 B1**
(45) Date of publication and mention of the grant of the patent: **29.10.2025**
(21) Application number: 21786117.8
(22) Date of filing: 27.09.2021
(51) Int. Cl.: A61F 2/89, A61F 2/07, A61F 2/88, A61F 2/915

(54) **IMPLANT AND METHOD OF MAKING AN IMPLANT**
IMPLANTAT UND VERFAHREN ZUR HERSTELLUNG EINES IMPLANTATS
IMPLANT ET SON PROCÉDÉ DE FABRICATION

(43) Date of publication of application: 07.08.2024
(73) Proprietor: Angiomed GmbH & Co. Medizintechnik KG, 76227 Karlsruhe (DE)
(72) Inventor: ZICKWOLF, Sebastian, 76227 Karlsruhe (DE); SCHROEER, Martin, 76227 Karlsruhe (DE)
(74) Representative: Hoffmann Eitle
(86) International application number: PCT/EP2021/076466
(87) International publication number: WO 2023/046301

(56) References cited:
- EP-B1- 1 903 998
- WO-A1-2011/034796
- WO-A1-2018/211796
- CN-A- 106 466 694
- JP-A- 2011 067 282
- US-A1- 2003 144 725
- US-A1- 2020 375 767

## Description

### Technical Field

The present invention relates to implants, in particular stent and stent grafts (covered stents). It also relates to a method of making an implant.

### Technical Background

For a number of medical conditions, stents and stent grafts are being used as a treatment. One such condition is peripheral artery disease (PAD), which most commonly affects the arteries of the legs. The primary cause of PAD is atherosclerosis, which occurs when arterial inflammation, cholesterol, calcium and scar tissue build up, forming a plug that clogs the arteries and slows blood flow to the legs. The most common symptoms of PAD involving the lower extremities are cramping, pain or tiredness in the leg or hip muscles when walking or climbing stairs (claudication). For stenosis and occlusions in iliac arteries, primary stenting with balloon expandable and self-expanding stents is a widely accepted practice. However, covered stents have shown a patency benefit over bare stents in several clinical studies and are thus favoured.

One issue with covered stents is their flexibility. In order to be able to place a stent in tortuous vessels, it is necessary for that stent to be highly flexible. Thus, having a more flexible stent would be highly desirable since it would increase the field of application of such stents.

WO 2011/034796 A1 discloses an implant with helical turns comprising struts connected in zig-zag pattern.

### Summary of the Invention

The present invention aims at alleviating or solving at least some of the problems mentioned above.

The invention is defined by the independent claims 1 and 13.

Embodiments are set out in the dependent claims.

According to claim 1, an implant having a base stent is provided that has a tubular shape and that surrounds a lumen that allows, when in use, blood to flow through. The base stent comprises a first section that has helical turns. The helical turns are connected by connectors that extend between adjacent helical turns and that limit the movement of the adjacent helical turns relative to each other. By "adjacent helical turns", it is meant that the helical turns of the first section of the base stent are adjacent to each other along the longitudinal direction of the base stent. Put differently, by them being adjacent to each other, there is no further helical turn between two adjacent helical turns. The helical turns comprise struts that are arranged in a zig-zag pattern, where the individual struts are connected at apices to form that pattern. At those apices, the individual legs of the zig-zag pattern change direction.

The apices of the adjacent helical turns between at least some, in embodiments all, adjacent helical turns that are oriented towards the respectively other one of the adjacent helical turns (that is, the inner apices), when viewed along the axial direction, and the two connectors define a shape. This shape is such that the longitudinal dimension of that shape is, at the positions of the two connectors, smaller than at an intermediate position between those two connectors. In embodiments, the shape, which is a polygon, is such that the longitudinal dimension steadily increases when moving from one of the connectors to the point of maximum axial dimension and then decreases steadily when moving to the respective other connector. The shape that is defined by the inner apices and by the two connectors is obtained by connecting the end points of one of the connectors with straight lines with the respective nearest one of the inner apices of the adjacent helical turns in the direction towards the respective other connector. From those respective inner apices, the next inner apex in the direction towards the other connector is connected with a straight line, until finally, the last such inner apex has been reached, which is then connected with a straight line with the nearest end of the other connector. The shape defined by the inner apices and by the two connectors improves the flexibility of the base stent. When the stent is bent, the helical turns will be constrained in their movement at the connectors. However, the stent will be able to move more easily at positions between those connectors. Accordingly, when the stent is bent, a larger relative movement between adjacent helical turns occurs at those positions. By having the shape as defined previously, which is, according to the invention, a roughly hexagonal shape (according to other configurations, not covered by the invention, the shape can be a diamond shape), a larger degree of motion become possible, thus increasing flexibility. In particular, it becomes less likely that at those positions where the highest degree of movement, the struts and the apices of one helical turn interfere with those of an adjacent helical turn. Since such an interference encumbers the flexibility of the stent, having the previously described shape is advantageous in improving the stent flexibility. It is worth noting that, in some embodiments, the highest axial dimension of the shape is between those apices at or nearest to the geometric centre of the shape when moving from one connector to the adjacent connector. Since the movement of the adjacent apices at this position is highest, one also needs the highest axial distance at that point to guarantee an optimal flexibility.

In embodiments, the base stent is covered by a covering material that covers at least parts of the luminal and/or abluminal surface of the base stent so as to form a stent graft. Any suitable covering material can be used. In particular, ePTFE, PET (polyethylene terephthalate), and PU (polyurethane) are envisaged. Such stent grafts are particularly good for treating PAD. In other embodiments, the base stent is not covered by a covering material so that the implant can be considered a stent, as compared with a stent graft.

In embodiments, in the unconstrained state of the implant, the struts extend inside a cylindrical surface of the stent. Put differently, the struts are arranged so that they (and, in embodiments, also the apices) are arranged within a cylindrical surface so that they do not extend inwardly or outwardly. If a covering material is used, since the covering material does not need to cover any such inwardly or outwardly extending struts or apices, the thickness of that covering material can be reduced. Furthermore, and rather generally, the base stent assumes a lower profile, which is generally advantageous.

In further embodiments, the first axial dimension is at least 105%, in further embodiments at least 120%, of the larger one of the second axial dimensions. By having such a relationship between the first and the larger one of the second axial dimensions, a significant difference in the length is ensured which leads to an overall greater flexibility since the difference between those dimensions is sufficiently large to allow for a good flexibility.

In embodiments, the first section is arranged at a central part of the base stent. There are additionally at least one, in embodiments two, second sections arranged adjacent to and connected to the first section which are configured by struts that are arranged in a ring-like shape. Having such second sections made of ring-like portions improves the stability of the implant.

In embodiments, the implant further comprises radiopaque markers at one, and further embodiments both, longitudinal ends. Such markers allow for a visualisation of the stent when placing it and thus aid the surgeon.

It is according to embodiments that the markers are essentially rectangular, with an axis of the rectangle in embodiments being arranged along the axial direction of the unconstrained implant. By the unconstrained implant, an implant is meant that is not being bent by an external force. Since the markers are essentially rectangular, they can be very space efficiently arranged one adjacent to the other, in contrast to, for example, circular makers. Accordingly, the overall area can be increased which makes is easier to visualize them whilst placing the implant. In embodiments, the markers are made of or comprise tantalum or gold. If the axis of the rectangle is arranged along the axial direction of the unconstrained implant, it becomes possible to pack the markers closely with a particularly high efficiency also in the crimped state on an implant delivery system. Further, the area of the markers can be maximized, which makes them easier to locate during surgery.

In embodiments, the base stent comprises a self-expanding material. Such stents are particularly easy to deploy in a patient's body and are hence used for a variety of implants, such as stent grafts and stents.

According to another embodiment of the invention, at least some of the apices, in embodiments all of them, have a thickness along the axial direction of the unconstrained implant that is greater than or equal to a thickness of the struts connected to those apices, when measured along a direction perpendicular to the direction of extension of the struts between two neighbouring apices. Such a design reduces the stress that is applied inside the apex when bending the implant, which increases the service life of those apices and hence the service life of the implant.

In that context, in embodiments, the thickness of at least some, in embodiments all, of the apices along the axial direction of the unconstrained implant is less than 120% of the thickness of the struts connected to those apices, where the thicknesses are defined as set out in the preceding paragraph. It has been shown that having a higher thickness of more than 120% does not lead to a significant reduction in stress and may also hamper the flexibility of the implant.

In embodiments, the entirety of the base stent is covered with the covering material. This increases the range over which a thus obtained stent graft can be used for treating diseased blood vessels.

In further embodiments, the covering material comprises ePTFE. ePTFE is highly biocompatible and is thus of particular use in stent grafts. Alternatively or additionally, PET and PU can be used.

According to another aspect of the invention, the invention relates to a method of making an implant as previously defined. According to that aspect, an implant precursor is provided. This implant precursor is an object that will be turned into an implant ready for implantation. In embodiments, the implant precursor could be a laser cut tube. The implant precursor comprises struts arranged in helical turns, with adjacent helical turns being connected by connectors.

The implant precursor is arranged on a mandrel to thus shape the implant precursor so that the shape that is defined by the inner apices of two axially adjacent helical turns and by two connectors that are adjacent to one another has, in the unconstrained state of the implant, at a position between the two connectors delimiting it, a longer first axial dimension than a second axial dimensions at the positions of the two connectors. Accordingly, it assumes the previously described diamond shape. The mandrel can, in embodiments, take the form of a cylinder that has engraved on its outer surface grooves that are arranged for retaining and holding the struts and connectors of the implant precursor. In other embodiments, it comprises pins on its outside to retain the implant precursor in shape.

In embodiments, the implant precursor comprises a shape memory material. Further, the implant precursor, when arranged on the mandrel, is heated above the phase transition temperature of the shape memory material to thereby set the hysteresis information in the shape memory material so that the shape memory material will later on be arranged to recover the shape of the implant precursor when it is arranged on the mandrel. The shape memory material, which, in embodiments is nitinol, has thus programmed into it the shape that is set on the mandrel and will thus recover it. If, as is the case in embodiments, the transition temperature of the shape memory material is at or slightly below body temperature, such an implant will naturally recover its preset shape when being introduced into a patient's body.

In embodiments, the mandrel comprises release grooves arranged so as to extend underneath the implant precursor. The implant precursor is released from the mandrel using tools introduced into the release grooves to thus aid in lifting off the implant precursor. Such release grooves make removing the implant precursor easier.

### Brief Description of the Drawings

Figure 1 shows a base stent to be used in a stent graft according to a first embodiment of the invention.
Figure 2 shows a conventional stent graft (Figure 2a)) and a stent graft according to the invention (Figure 2b)).
Figure 3 illustrates the differences between conventional and inventive stent grafts.
Figure 4 shows stress profiles in the apices of different stent grafts.
Figure 5 shows a mandrel used for shaping the base stent according to the first embodiment.
Figures 6a) and b) show steps in the shaping of the base stent according to the first embodiment.
Figure 7 shows steps that can be taken during the manufacture of the base stent according to the first embodiment.

### Detailed Description of the Drawings

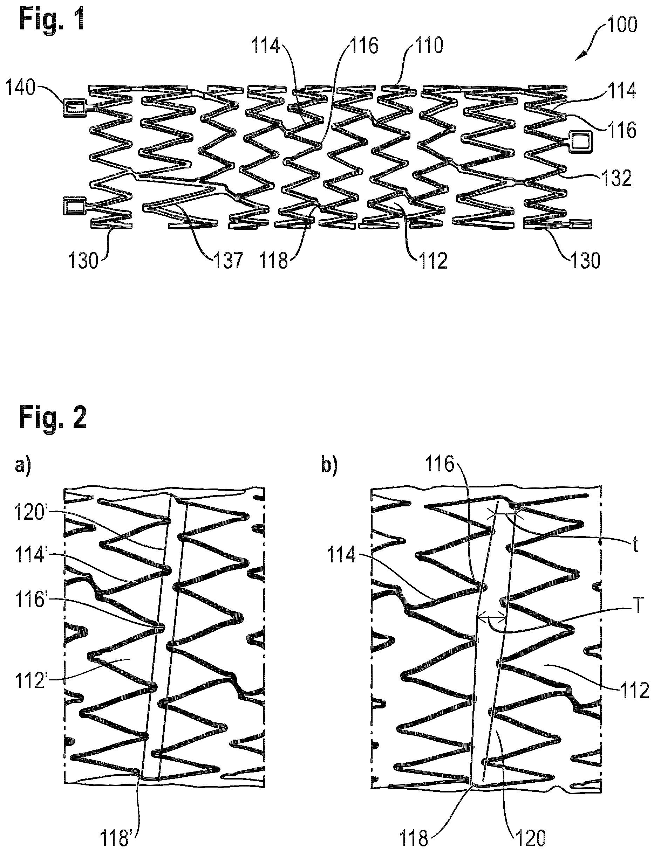

Figure 1 shows a base stent 100 to be used in a stent graft according to the present invention. As can be seen from that figure, a base stent 100 comprises a first section 110 and two second sections 130 arranged at either longitudinal end of the first section 110, where both the first section 110 and the second sections 130 have a tubular shape. The first section 110 and the second sections 130 are connected by struts 137 that are longer than the struts that constitute the first and the second sections 110, 130.

The first section 110 comprises struts 114 that are arranged in helical turns 112 extending around the longitudinal axis of the base stent 100, with adjacent helical turns 112 being connected by connectors 118. The struts 114 are arranged in a zig-zagging pattern and change their direction at apices 116.

The second sections 130 are also formed of zig-zagging struts 114 connected at apices 116. However, in contrast with the helical shape of the first turns 112, the struts 114 of the first sections create a ring-like turn, rather than a helix. At the respective longitudinal ends of the second section 130, eight radiopaque markers 140 are arranged, with four markers 140 being provided at either end. The number of markers 140 is non-limiting and just serves as an example. There are presently 33 struts 114 per winding, and a there is a connector 118 after five pairs of struts 114, i.e., after ten struts 114. Again, those numbers are non-limiting. For an improved flexibility, the struts 114 are arranged according to a peak to valley design, in addition to the structure to be discussed below. The radiopaque markers can be made of any suitably radiopaque material, such as tantalum, gold, or platinum-iridium alloys. In addition, different concepts of attaching the markers, such as markers that are encapsulated in the covering material are possible.

Figure 2 shows how the present invention differs from the prior art. Whilst Figure 2a) shows the structure of the struts according to a prior art design, Figure 2b) shows an inventive design.

Starting with Figure 2a), it can be seen that when going from one connector 118' to another adjacent connector 118' that both connect two helical turns 112', and when connecting with a straight line the inner apices 116' that connect the individual struts 114' of adjacent helical windings, a line 120' is arrived at that is, when viewed in the cylindrical surface of the base stent, straight.

A different assessment holds when reviewing the structure according to the invention as shown in Figure 2b). When again moving from one connector 118 to the next connector 118 connecting between the same two adjacent helical turns 112, and when connecting with straight lines the innermost apices 116, a bent line 120 is arrived at that first bends outwardly and then bends inwardly when moving from one of the connectors 118 to the respective other connector 118. This is the case for both adjacent helical windings 112. As pointed out before, such a design improves the flexibility of the resulting stent graft. In the example that is shown in Fig. 2b), the width t at the ends is, in the unconstrained state, 0.470mm, whilst the width T at the center is 0.678mm, which corresponds to an increase by 44%. It is to be noted that these values are just specific examples and should not be construed as limiting.

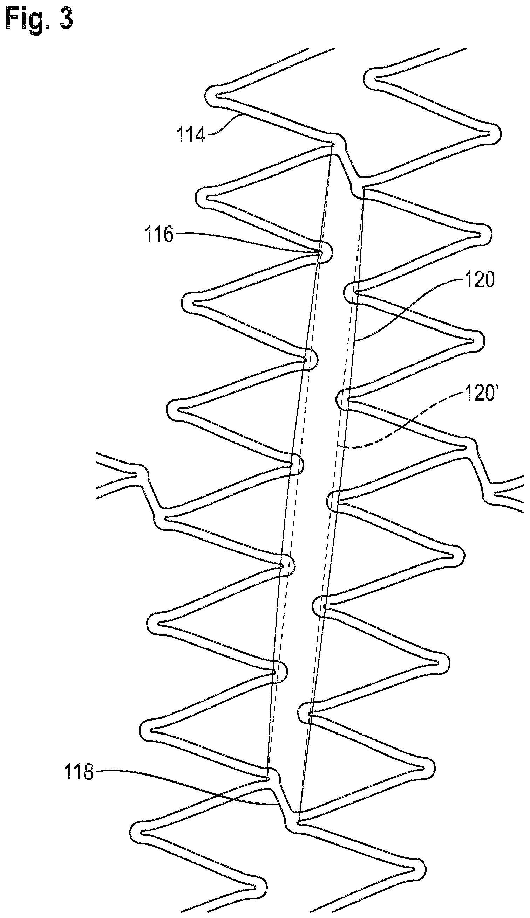

These structures are juxtaposed in Figure 3. As can be seen from that juxtaposition, where both the connecting line 120 according to the present invention and the connecting line 120' according to the prior art are shown, the connecting line 120 according to the present invention bulges out at the middle position between the two connectors 118 and thus leads to a higher degree of flexibility. In line with what was described previously, the connecting line 120 has been drawn so as to connect the inner apices 116 between adjacent connectors 118. Adjacent connectors hereby mean connectors that are arrived at when moving along the direction of the helical winding.

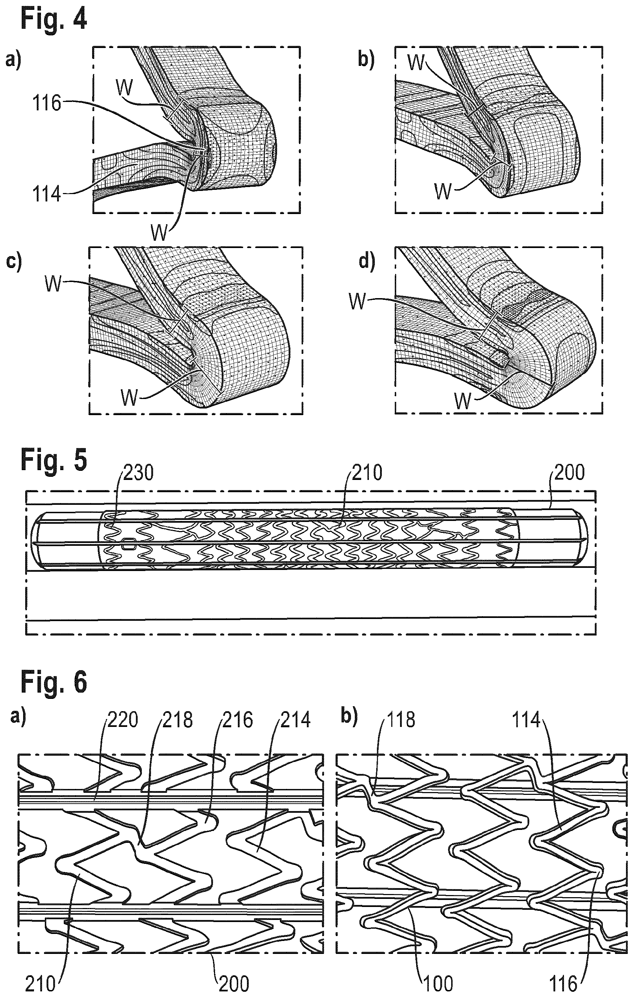

Figure 4 shows stress profiles according to different embodiments of the present invention. In those figures, the stress profile in the apex 116 with connected struts 114 can be seen. Figure 4a shows an apex thickness W of 0.084mm which is smaller than the strut thickness w by 0.01mm. Figure 4b) shows a situation where the apex thickness W is the same as the strut thickness w. Figure 4c) shows a case where an apex thickness W of 0.104mm is greater than the strut thickness w by 0.02mm. Figure 4d) shows a case where the apex thickness W of 0.124mm is significantly larger than the strut thickness w by 0.03mm. As can be seen from going from Figure 4a) to 4b) and 4c), the stress in the apex is reduced, which improves the service life of the base stent. However, when going from the stress profile of 4c) to 4d), the stress does not decrease even more. Accordingly, it can be seen that, whilst having an apex thickness W being the same or slightly larger than the strut thickness w is advantageous, so as to reduce stress, the effect does not continue with every increasing apex thicknesses W.

Figure 5 shows a mandrel 200 used for shaping the base stent 100. Aspects of the mandrel 200 are also illustrated in Figures 6a), b). The mandrel 200 has a cylindrical form and comprises grooves on its periphery that serve to accommodate the base stent 100 to be shaped during the shaping process. In more detail, the grooves comprise a first groove section 210 for retaining the struts 214 of the first section 110 of the base stent 100 and two second groove sections 230 for retaining the struts of the second sections 130 of the base stent 100. The first groove section 210 and the second groove sections 230 comprise (cf. Fig. 6a)) connector grooves 218 for retaining connectors 118 of the base stent 100, strut grooves 214 for retaining struts 114 of the base stent 100, and apex grooves 216 for retaining apices 116 of the base stent 100.

The mandrel 200 also comprises release grooves 220 that are recessed more deeply into the surface of the mandrel 200 than the grooves 218, 216, 214 for retaining the base stent 100. The release grooves 220 extend axially along the longitudinal axis of the mandrel 200.

The connector grooves 218, strut grooves 214, and apex grooves 216 are arranged so that a correspondingly shaped base stent 100 can be fitted into them and retained by them when shaping the base stent 100.

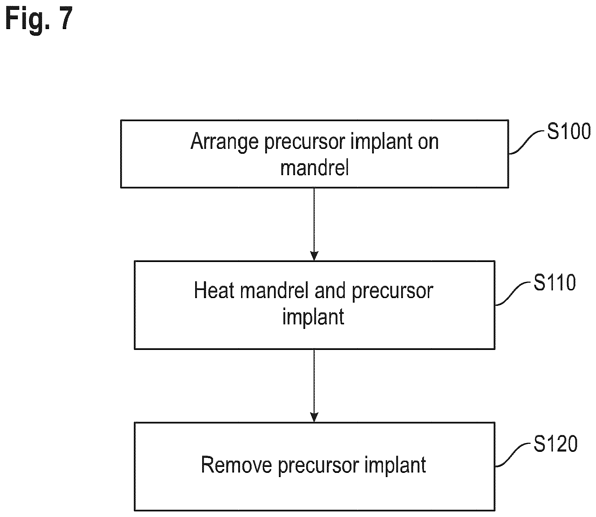

Accordingly, and as illustrated schematically in Figure 7, during manufacture, base stent 100 is produced, typically from a shape memory alloy (such as nitinol), by cutting from a tube of raw material. The retained material is then fitted into and retained by those grooves 214, 216, 218, as can be seen from Fig. 6b) (step S100).

Subsequently, the assembly of the base stent 100 and the mandrel 200 is heated to a sufficiently high temperature to set the material of the base stent 100 (step S110). It is then cooled down, and the base stent 100 is removed from the mandrel 200 (if necessary by introducing tools into the release grooves 220 to lift it off) (step S120).

The base stent 100 is treated further (which could, potentially, include a step of covering it if a stent graft is to be manufactured) and is then crimped down and loaded onto a delivery platform for implantation in a patient.

It is the grooves 214, 216, 218 of the mandrel 200 that impart the diamond structure on the base stent 100. That is, those grooves 214, 216, 218 ensure that the diamond structure as illustrated in Figure 3 is achieved by making sure that the connecting lines 120 bulge outwardly. Accordingly, the diamond structure is, for a base stent 100 comprising a shape memory material, the diamond structure is present in the hysteresis information regarding the final shape that has been set during manufacture.

## Claims

1. Implant, comprising:
a base stent (100) having a tubular shape and surrounding a lumen, the base stent comprising a first section (110) having helical turns (112), wherein adjacent helical turns (112) are connected to each other by connectors (118) that extend between adjacent helical turns (112) and that limit movement of the adjacent helical turns (112) relative to each other,
wherein the helical turns comprise struts (114) that are connected in a zig-zag pattern, with individual struts being connected at apices (116), and
wherein the shape that is defined by the inner apices of two axially adjacent helical turns (112) and by two connectors (118) that are adjacent to one another is roughly hexagonal and has, in the unconstrained state of the implant, at a position between the two connectors delimiting it, a longer first axial dimension (T) than second axial dimensions (t) at the positions of the two connectors (118).

2. Implant according to claim 1,
the implant further comprising a covering material that covers at least parts of the luminal and/or abluminal surface of the base stent (100).

3. Implant according to claim 1 or 2, wherein in the unconstrained state of the implant, the struts are arranged within a cylindrical surface of the implant so that they do not extend inwardly or outwardly, with the apices (116) arranged within the cylindrical surface.

4. Implant according to one of the preceding claims, wherein the first axial dimension is at least 105%, optionally more than 120% of the larger one of the second axial dimensions.

5. Implant according to one of the preceding claims, wherein the first section (110) is arranged at a central part of the base stent, the base stent further comprising one, optionally two second sections (130) that are adjacent to the first section (110), the second sections being configured by struts (132) that are arranged in a ring-like shape.

6. Implant according to one of the preceding claims, further comprising radiopaque markers (140) at one, optionally both, longitudinal ends.

7. Implant according to claim 6, wherein the markers (140) are essentially rectangular, with an axis of the rectangle optionally being along the axial direction of the unconstrained implant.

8. Implant according to one of the preceding claims, the base stent comprising a self-expanding material.

9. Implant according to one of the preceding claims, wherein at least some of the apices (116) have a thickness (W) along the axial direction of the unconstrained implant that is greater than or equal to a thickness (w) of the struts (114) connected to those apices (116) when measured along a direction perpendicular to a direction of extension of the struts.

10. Implant according to claim 9, wherein the thickness of at least some, optionally all of the apices along the axial direction of the unconstrained implant is less than 120% of the thickness of the struts connected to those apices.

11. Implant according to one of claims 2 to 10, when comprising the features of claim 2, wherein the entirety of the base stent is covered with the covering material.

12. Implant according to one of claims 2-11, when comprising the features of claim 2, wherein the covering material comprises ePTFE, PET, and/or PU.

13. Method of making an implant, the method being arranged for making an implant according to one of the preceding claims, the method comprising:
- providing an implant precursor, the implant precursor comprising struts (112) arranged in helical turns, adjacent helical turns being connected by connectors (118),
- arranging the implant precursor on a mandrel (200) to thus shape the implant precursor so that the shape that is defined by the inner apices of two axially adjacent helical turns (112) and by two connectors (118) that are adjacent to one another is roughly hexagonal and has, in the unconstrained state of the implant, at a position between the two connectors (118) delimiting it, a longer first axial dimension (T) than second axial dimensions (t) at the positions of the two connectors (118).

14. Method of claim 13, wherein the implant precursor comprises a shape memory material and wherein the implant precursor, when arranged on the mandrel (200), is heated above the phase transition temperature of the shape memory material to thereby set the hysteresis information in the shape memory material so that the shape memory material will later on be arranged to recover the shape of the implant precursor when it is arranged on the mandrel (200).

15. Method of claim 13 or 14, wherein the mandrel (200) comprises release grooves (220) arranged so as to extend underneath the implant precursor, the implant precursor being released from the mandrel (200) using tools introduced into the release grooves (220) to thus aid in lifting off the implant precursor.

## Patentansprüche

1. Implantat, umfassend:
einen Basisstent (100), der eine röhrenförmige Gestalt aufweist und ein Lumen umgibt, der Basisstent umfassend einen ersten Abschnitt (110) mit spiralförmigen Windungen (112), wobei benachbarte spiralförmige Windungen (112) durch Anschlüsse (118) miteinander verbunden sind, die sich zwischen benachbarten spiralförmigen Windungen (112) erstrecken und die Bewegung der benachbarten spiralförmigen Windungen (112) relativ zueinander begrenzen,
wobei die spiralförmigen Windungen Streben (114) umfassen, die in einem Zickzackmuster verbunden sind, wobei einzelne Streben an Scheitelpunkte (116) verbunden sind, und
wobei die Form, die durch die inneren Scheitelpunkte zweier axial benachbarter spiralförmiger Windungen (112) und durch zwei Anschlüsse (118) definiert wird, die zueinander benachbart sind, ist ungefähr sechseckig und weist im unbelasteten Zustand des Implantats an einer Position zwischen den beiden an ihr begrenzenden Anschlüssen eine längere erste axiale Abmessung (T) auf als zweite axiale Abmessungen (t) an den Positionen der beiden Anschlüsse (118).

2. Implantat nach Anspruch 1,
wobei das Implantat weiter umfassend ein Abdeckmaterial, das zumindest Teile der luminalen und/oder abluminalen Oberfläche des Basisstents (100) bedeckt.

3. Implantat nach Anspruch 1 oder 2, wobei die Streben im unbelasteten Zustand des Implantats innerhalb einer zylindrischen Oberfläche des Implantats angeordnet sind, sodass sie sich weder nach innen noch nach außen erstrecken, wobei die Scheitelpunkte (116) innerhalb der zylindrischen Oberfläche angeordnet sind.

4. Implantat nach einem der vorstehenden Ansprüche, wobei die erste axiale Abmessung mindestens 105 %, optional mehr als 120 % der größeren der zweiten axialen Abmessungen beträgt.

5. Implantat nach einem der vorstehenden Ansprüche, wobei der erste Abschnitt (110) in einem zentralen Teil des Basisstents angeordnet ist, der Basisstent weiter umfassend einen, optional zwei, zweite Abschnitte (130), die an den ersten Abschnitt (110) angrenzen, wobei die zweiten Abschnitte durch Streben (132) konfiguriert sind, die in einer ringartigen Form angeordnet sind.

6. Implantat nach einem der vorstehenden Ansprüche, weiter umfassend röntgendichte Marker (140) an einem oder optional beiden Längsenden.

7. Implantat nach Anspruch 6, wobei die Marker (140) im Wesentlichen rechteckig sind, wobei eine Achse des Rechtecks optional entlang der axialen Richtung des unbeschränkten Implantats verläuft.

8. Implantat nach einem der vorstehenden Ansprüche, der Basisstent umfassend ein selbstexpandierendes Material.

9. Implantat nach einem der vorstehenden Ansprüche, wobei zumindest einige der Scheitelpunkte (116) entlang der axialen Richtung des unbeschränkten Implantats eine Dicke (W) aufweisen, die größer oder gleich einer Dicke (w) der Streben (114) ist, die mit diesen Scheitelpunkten (116) verbunden sind, wenn sie entlang einer Richtung senkrecht zu einer Erstreckungsrichtung der Streben gemessen wird.

10. Implantat nach Anspruch 9, wobei die Dicke von zumindest einigen, optional allen Scheitelpunkte entlang der axialen Richtung des unbeschränkten Implantats weniger als 120 % der Dicke der mit diesen Scheitelpunkte verbundenen Streben beträgt.

11. Implantat nach einem der Ansprüche 2 bis 10, wenn umfassend die Merkmale des Anspruchs 2, wobei der gesamte Basisstent mit dem Abdeckmaterial bedeckt ist.

12. Implantat nach einem der Ansprüche 2 - 11, wenn umfassend die Merkmale des Anspruchs 2, wobei das Abdeckmaterial ePTFE, PET und/oder PU umfasst.

13. Verfahren zur Herstellung eines Implantats, wobei das Verfahren zur Herstellung eines Implantats nach einem der vorhergehenden Ansprüche ausgelegt ist, das Verfahren umfassend:
- Bereitstellen eines Implantatvorläufers, der Implantatvorläufer umfassend Streben (112), die in spiralförmigen Windungen angeordnet sind, wobei die benachbarten spiralförmigen Windungen durch Anschlüsse (118) verbunden sind,
- Anordnen des Implantatvorläufers auf einem Dorn (200), um so den Implantatvorläufer zu formen, sodass die Form, die durch die inneren Scheitelpunkte zweier axial benachbarter spiralförmiger Windungen (112)
und durch zwei nebeneinander liegende Anschlüsse (118) definiert ist, die etwa sechseckig ist und im ungespannten Zustand des Implantats an einer Stelle zwischen den beiden an ihn begrenzenden Anschlüssen (118) eine längere erste Axialabmessung (T) als zweite Axialabmessungen (t) an den Stellen der beiden Anschlüsse (118) aufweist.

14. Verfahren nach Anspruch 13, wobei der Implantatvorläufer ein Formgedächtnismaterial umfasst und wobei der Implantatvorläufer, wenn er auf dem Dorn (200) angeordnet ist, über die Phasenübergangstemperatur des Formgedächtnismaterials erhitzt wird, um dadurch die Hystereseinformationen im Formgedächtnismaterial einzustellen, sodass das Formgedächtnismaterial später so angeordnet wird, dass es die Form des Implantatvorläufers wiedererlangt, wenn dieser auf dem Dorn (200) angeordnet wird.

15. Verfahren nach Anspruch 13 oder 14, wobei der Dorn (200) Freigabenuten (220) umfasst, die so angeordnet sind, dass sie sich unterhalb des Implantatvorläufers erstrecken, wobei der Implantatvorläufer unter Verwendung von Werkzeugen, die in die Freigabenuten (220) eingeführt werden, vom Dorn (200) freigegeben wird, um so das Abheben des Implantatvorläufers zu erleichtern.

## Revendications

1. Implant, comprenant :
un stent de base (100) présentant une forme tubulaire et entourant une lumière, le stent de base comprenant une première section (110) présentant des spires hélicoïdales (112), dans lequel des spires hélicoïdales (112) adjacentes sont reliées entre elles par des raccords (118) qui s'étendent entre des spires hélicoïdales (112) adjacentes et qui limitent le mouvement des spires hélicoïdales (112) adjacentes les unes par rapport aux autres,
dans lequel les spires hélicoïdales comprennent des entretoises (114) qui sont reliées selon un motif en zigzag, des entretoises individuelles étant reliées aux sommets (116), et
dans lequel la forme qui est définie par les sommets internes de deux spires hélicoïdales (112) axialement adjacentes et par deux raccords (118) qui sont adjacents l'un à l'autre est approximativement hexagonale et présente, à l'état non contraint de l'implant, en une position entre les deux raccords la délimitant, une première dimension axiale (T) plus longue que des secondes dimensions axiales (t) aux positions des deux raccords (118).

2. Implant selon la revendication 1,
l'implant comprenant en outre un matériau de revêtement qui recouvre au moins certaines parties de la surface luminale et/ou abluminale du stent de base (100).

3. Implant selon la revendication 1 ou la revendication 2, dans lequel, à l'état non contraint de l'implant, les entretoises sont agencées à l'intérieur d'une surface cylindrique de l'implant de sorte qu'elles ne s'étendent ni vers l'intérieur ni vers l'extérieur, les sommets (116) étant agencés à l'intérieur de la surface cylindrique.

4. Implant selon l'une des revendications précédentes, dans lequel la première dimension axiale équivaut à au moins 105 %, éventuellement à plus de 120 % de la plus grande des secondes dimensions axiales.

5. Implant selon l'une des revendications précédentes, dans lequel la première section (110) est agencée au niveau d'une partie centrale du stent de base, le stent de base comprenant en outre une, éventuellement deux secondes sections (130) qui sont adjacentes à la première section (110), les secondes sections étant configurées par des entretoises (132) qui sont agencées en forme d'anneau.

6. Implant selon l'une des revendications précédentes, comprenant en outre des repères radio-opaques (140) à une, éventuellement aux deux, extrémité(s) longitudinale(s).

7. Implant selon la revendication 6, dans lequel les repères (140) sont sensiblement rectangulaires, un axe du rectangle étant éventuellement situé le long de la direction axiale de l'implant non contraint.

8. Implant selon l'une des revendications précédentes, le stent de base comprenant un matériau auto-expansible.

9. Implant selon l'une des revendications précédentes, dans lequel au moins certains des sommets (116) présentent une épaisseur (W) le long de la direction axiale de l'implant non contraint qui est supérieure ou égale à une épaisseur (w) des entretoises (114) reliées à ces sommets (116) lorsqu'elles sont mesurées le long d'une direction perpendiculaire à une direction d'extension des entretoises.

10. Implant selon la revendication 9, dans lequel l'épaisseur d'au moins certains, éventuellement de tous les sommets le long de la direction axiale de l'implant non contraint représente moins de 120 % de l'épaisseur des entretoises reliées à ces sommets.

11. Implant selon l'une des revendications 2 à 10, lorsqu'il comprend les caractéristiques de la revendication 2, dans lequel l'ensemble du stent de base est recouvert du matériau de revêtement.

12. Implant selon l'une des revendications 2 à 11, lorsqu'il comprend les caractéristiques de la revendication 2, dans lequel le matériau de revêtement comprend de l'ePTFE, du PET et/ou du PU.

13. Procédé de fabrication d'un implant, le procédé étant agencé pour fabriquer un implant selon l'une des revendications précédentes, le procédé comprenant :
- la fourniture d'un précurseur d'implant, le précurseur d'implant comprenant des entretoises (112) agencées en spires hélicoïdales, des spires hélicoïdales adjacentes étant reliées par des raccords (118),
- l'agencement du précurseur d'implant sur un mandrin (200) pour ainsi façonner le précurseur d'implant de sorte que la forme qui est définie par les sommets internes de deux spires hélicoïdales (112) axialement adjacentes et par deux raccords (118) qui sont adjacents l'un à l'autre soit approximativement hexagonale et présente, à l'état non contraint de l'implant, en une position entre les deux raccords (118) la délimitant, une première dimension axiale (T) plus longue que des secondes dimensions axiales (t) aux positions des deux raccords (118).

14. Procédé selon la revendication 13, dans lequel le précurseur d'implant comprend un matériau à mémoire de forme et dans lequel le précurseur d'implant, lorsqu'il est agencé sur le mandrin (200), est chauffé au-dessus de la température de transition de phase du matériau à mémoire de forme pour ainsi fixer les informations d'hystérésis dans le matériau à mémoire de forme de sorte que le matériau à mémoire de forme sera ultérieurement agencé pour reprendre la forme du précurseur d'implant lorsqu'il est agencé sur le mandrin (200).

15. Procédé selon la revendication 13 ou la revendication 14, dans lequel le mandrin (200) comprend des rainures (220) de libération agencées de manière à s'étendre sous le précurseur d'implant, le précurseur d'implant étant libéré du mandrin (200) en utilisant des outils introduits dans les rainures (220) de libération pour ainsi faciliter le soulèvement du précurseur d'implant.
